# NEUE EUROPÄISCHE PATENTSCHRIFT

(11) **EP 2 723 312 B2**
(45) Veröffentlichungstag und Bekanntmachung des Hinweises auf die Entscheidung über den Einspruch: **18.03.2020**
(45) Hinweis auf die Patenterteilung: 04.10.2017
(21) Anmeldenummer: 12727667.3
(22) Anmeldetag: 15.06.2012
(51) Int. Cl.: A61K 8/73, A61K 8/895, A61Q 17/04, A61K 8/06

(54) **TAPIOKASTÄRKE IN SILIKONELASTOMER-HALTIGEN KOSMETISCHEN ZUBEREITUNGEN**
TAPIOCA STARCH IN COSMETIC PREPARATIONS CONTAINING SILICONE ELASTOMER
AMIDON DE TAPIOCA UTILISÉ DANS DES PRÉPARATIONS COSMÉTIQUES CONTENANT DES ÉLASTOMÈRES DE SILICONE

(30) Priorität: 27.06.2011 DE 102011078100
(43) Veröffentlichungstag der Anmeldung: 30.04.2014
(73) Patentinhaber: Beiersdorf AG, 20253 Hamburg (DE)
(72) Erfinder: HEUER, Björn, 22145 Hamburg (DE); KÖHLER, Manuela, 22147 Hamburg (DE); CEMIN, Luciana, 21075 Hamburg (DE)
(74) Vertreter: Wihstutz, Kolja
(86) Internationale Anmeldenummer: PCT/EP2012/061474
(87) Internationale Veröffentlichungsnummer: WO 2013/000738

(56) Entgegenhaltungen:
- EP-A1- 2 181 699
- EP-A2- 0 827 983
- EP-A2- 2 181 698
- EP-A2- 2 255 849
- WO-A2-2008/085360
- US-A- 4 894 222
- US-A- 5 654 362
- US-A- 5 945 090
- US-A- 6 024 944
- US-A1- 2004 044 121
- US-A1- 2004 228 888
- US-A1- 2005 277 768
- US-A1- 2006 034 876
- US-A1- 2010 322 877
- DATABASE GNPD [O] 31 January 2009 (2009-01-31), BEIERSDORF: "Nivea Facial Sunblock", retrieved from Mintel Database accession no. 1036175
- DATABASE GNPD [O] 31 May 2011 (2011-05-31), BEIERSDORF: "Nivea Sun Kids - Sun Lotion", retrieved from Mintel Database accession no. 1548109
- NATIONAL STARCH PERSONAL CARE: "Naviance Tapioca certified organic biopolymer INCI: Tapioca Starch (proposed) 28-053A (US organic certification) 28-093A (European organic certification)", PRODUKTINFORMATION, 4 September 2008 (2008-09-04), pages 1 - 5

## Beschreibung

Die vorliegende Erfindung betrifft eine kosmetische Zubereitung mit UV-Filtern, Silikonelastomergelen und Tapiokastärke.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut. Um diese jung und makellos zu halten werden seit vielen Jahren Hautpflegeprodukte eingesetzt.

Anwender stellen an moderne Hautpflegeprodukte, speziell in Form von Cremes für die Anwendung im Gesicht, eine Reihe von Anforderungen. Sie sollen die Haut pflegen, insbesondere die Haut über einen langen Zeitraum mit Feuchtigkeit versorgen. Dieses wird in der Regel über "Moisturizer" realisiert, die Wasser im Stratum Corneum binden (z.B. Glycerin), zudem werden mit zugesetzten Lipiden die oberen Hautschuppen geschmeidig gehalten und der transepidermale Wasserverlust reduziert. Desweiteren erwarten die Anwender häufig eine Wirkung gegen die Anzeichen von Hautalterung, wie z.B. die Ausbildung von Falten, das Nachlassen der Hautelastizität oder die Entstehung von Altersflecken. Hierzu werden zum einen Wirkstoffe verwendet, die beispielsweise die Aktivität der Hautzellen stimulieren, aber auch verstärkt UV-Filter eingesetzt, die besonders die für die Hautalterung verantwortlichen UVA-Strahlen blockieren. Alle diese Inhaltsstoffe stellen eine große Herausforderung für eine angenehme Sensorik dar, da sie nach dem Verdunsten der flüchtigen Bestandteile der Formulierung als Film auf der Haut zurückbleiben, und dort sensorisch vom Verbraucher wahrgenommen werden. Die fühlbaren Rückstände werden dabei oft negativ beschrieben und verhindern das Gefühl dass das Produkt komplett in die Haut "eingezogen" sei.

Um die durch die Formulierung und die Marktanforderungen benötigten Rückstände sensorisch attraktiver zu machen, wird eine Reihe von Sensorik-Additiven eingesetzt. Insbesondere Silikonverbindungen und Puderrohstoffe sind hierbei zu nennen, da sie das klebrige Gefühl von z.B. Glycerin oder die ölig/fettige Anmutung vieler Lipide oder UV-Filter maskieren können. Man findet in Produkten verstärkt den Einsatz von Silikonelastomeren, welches verschieden vernetzte und/oder modifizierte Polydimethylsiloxane sind, die in der Regel in einem Medium als Gel gequollen vorliegen. Dieses Medium ist bevorzugt ein niedrigviskoses, bevorzugt auch flüchtiges Silikon. Diese Rohstoffklasse liefert ein pudrig/trockenes, samtig/seidiges Hautgefühl nach dem Verteilen und kann, je nach Formulierungstyp, ölig/fettig und/oder klebrige Rohstoffe maskieren.

Generell ist die angenehme Sensorik der Silikonelastomere in W/O Emulsionen stärker ausgeprägt als in O/W Emulsionen, da sie sich in diesem Fall in der äußeren, hydrophoben Phase aufhalten und somit unmittelbar sensorisch wahrgenommen werden können. Diese Formulierungen sind besonders reichhaltig und daher nicht für alle Verbraucher-Gruppen geeignet. Verbraucher, die eher normale bis Mischhaut haben, legen in der Regel deutlich weniger Wert auf ein reichhaltiges Produkt als Menschen mit trockener Haut. Das liegt besonders daran, dass die Reichhaltigkeit mit der Pflege und dem Schutz der Haut verbunden wird.

Für Verbraucher mit normaler bis Mischhaut werden fast ausschließlich die weniger reichhaltigen O/W Emulsionen eingesetzt. In diesen Formulierungen ist der Effekt der Silikonelastomere aber deutlich schwächer ausgeprägt, weswegen in der Regel eine recht hohe Einsatzkonzentration benötigt wird. Werden in einem Medium die Cyclopentasiloxan gequollene Elastomergele eingesetzt, bedeutet dies, dass ein hoher Anteil von eben Cyclopentasiloxan in der Formel vorhanden ist, was sensorisch für den Verbraucher merkbar ist. Die Formulierungen fühlen sich künstlich an, da das Silikon deutlich spürbar ist.

Es besteht Bedarf an einem leichten Gesichtspflegeprodukt für normale bis Mischhaut, welches mindestens einen Lichtschutzfaktor von 15 mit entsprechendem UVA-Schutz aufweist und bevorzugt mindestens 10% Glycerin enthält um den Verbrauchererwartungen in Bezug auf Schutz vor Hautalterung durch UV Strahlung und auf Feuchtigkeitsversorgung zu erfüllen. Das Produkt soll dabei schnell vollständig in die Haut einziehen und sich zu jeder Zeit natürlich anfühlen. Gewünschte Zusatznutzen sind z.B. eine effizient einstellbare Konsistenz zur Darreichung im Tiegel oder auch eine hohe Wasserfestigkeit, um die Produkteigenschaften z.B. auch beim Schwitzen oder anderen mit Wasser verbundenen Tätigkeiten zu behalten.

Nachteilig am Stande der Technik ist insbesondere der Umstand, dass die Wasserfestigkeit UV-Filter haltiger Zubereitungen meist nur durch den Einsatz von Filmbildnern (z.B. Polyvinylpyrrolidon) gewährleistet werden kann. Der Einsatz solcher Filmbildner führt jedoch zu einem klebrig unangenehmen Hautgefühl. Bei der Anwendung am Strand führen solche Zubereitungen darüber hinaus zu einer erhöhten Sandanhaftung. Silikonelastomergele, die das Hautgefühl positiv beeinflussen, können diesen Effekt allenfalls unzureichend kompensieren.

Es war daher die Aufgabe der vorliegenden Erfindung, die Wasserfestigkeit UV-Filter haltiger kosmetischer Zubereitungen zu erhöhen ohne (oder mit verringerter Einsatzkonzentration) auf die herkömmlichen Filmbildner zurückgreifen zu müssen. Dies gilt insbesondere für Silikonelastomergel haltige Zubereitungen.

Ein weiteres technisches Problem bei der Formulierung kosmetischer Zubereitungen stellt die Einstellung der für den Anwendungszweck geeigneten Viskosität der Zubereitung dar. Häufig müssen die Zubereitungen erst mit Hilfe polymerer Verdickungsmittel, beispielsweise mit Hilfe von Polyacrylaten, auf die gewünschte Viskosität gebracht werden. Derartige Verdickungsmittel sind jedoch häufig sensorisch unattraktiv und führen bei der Auftragung auf die Haut zur Röllchenbildung.

Es war daher die Aufgabe der vorliegenden Erfindung, die Viskosität kosmetischer Zubereitungen mit alternativen kosmetischen Inhaltsstoffen zu regulieren. Dies gilt insbesondere für UV-Filter und Silikonelastomergel haltige Zubereitungen.

Gelöst werden die Aufgaben durch eine kosmetische Zubereitung enthaltend gemäß Anspruch 1. Vorteilhafte Eigenschaften liegen auch in der Verwendung von Tapiokastärke in kosmetischen Zubereitungen, enthaltend UV-Filter und Silikonelastomere zur Erhöhung der Wasserfestigkeit, der Erhöhung der Viskosität.

Zwar kennt der Stand der Technik die EP 0893987, EP 1139997, EP 1551923, EP 1731136, EP 1784158, FR 2780642, FR 2862534, EP 0827983, US 2004/228888, US 2010/322877, US 4894222, US 2005/277768, US 2006/034876, US 2004/044121, US 5654362 sowie EP 1493421, doch konnten diese Schriften nicht den Weg zur vorliegenden Erfindung weisen. Außerdem kennt der Fachmann die EP 2181698, WO 2008/085360, US 6024944, EP 2255849, US 5945090, EP 2181699 sowie die Mintel GNPD Datenbank Einträge Nr. 1036175 und Eintrag Nr. 1548109, die ebenfalls nicht den Weg zur vorliegenden Erfindung weisen konnten.

Unter "erfindungsgemäß", "erfindungsgemäß vorteilhaft" etc. werden im Rahmen der vorliegenden Beschreibung Zubereitungen entsprechend der Ansprüche verstanden. Es ist erfindungsgemäß, wenn die Zubereitung in Form einer O/W-Emulsion vorliegt.

Vorteilhafte Ausführungsformen der vorliegenden Erfindung sind dadurch gekennzeichnet, dass die Zubereitung mindestens 5 Gew.-%, bezogen auf das Gesamtgewicht der Zubereitung, an UV-Filtern enthält.

Bei den erfindungsgemäß vorteilhaften Silikonelastomergelen handelt es sich um vernetzte, ggf. substituierte Organopolysiloxane, die in einem geeigneten Trägermedium gequollen sind. Die Vernetzung kann mittels aller gängigen Verfahren und Vernetzern durchgeführt worden sein. Trägermedien können z.B. cyclische oder lineare Silikone sowie organische Lipide sein.

Als erfindungsgemäße Siloxanelastomere kommen insbesondere die folgenden Elastomere als erfindungsgemäß vorteilhafte Ausführungsformen in Frage:
A) Siloxanelastomer, welches hergestellt wird durch das Hydrosilylierungs-Produkt von
   (1) einem linearen Alkenyl-Endgruppen aufweisenden Polyorganosiloxan der Formel:

      M^{vi}ₐD_{xD}^{vi}_{y}M₂₋ₐ

      worin x eine Zahl von 450 bis 1250, y eine Zahl von 0 bis 200, a eine Zahl von 0 bis 2 ist, unter der Einschränkung dass a+y im Bereich von 1 bis 20 liegt, wobei M^{vi} definiert ist als

      R¹R²R³SiO_{1/2}

      worin R¹ ein einwertiger ungesättigter Kohlenwasserstoffrest mit 2 Kohlenstoffatomen ist und R² und R³ jeweils unabhängig einwertige C₁bis C₄₀-Kohlenwasserstoffreste sind, wobei D definiert ist als

      R⁴R⁵SiO_{2/2}

      worin R⁴ und R⁵ jeweils unabhängig einwertige C₁₋₄₀-Kohllenstoffreste sind, wobei D^{vi} definiert ist als

      D^{vi}= R⁶R⁷SiO_{2/2}

      worin R⁶ ein einwertiger ungesättigter Kohlenwasserstoffrest mit 2 bis 10 Kohlenstoffatomen ist und R⁷ unabhängig ein einwertiger C₁₋₄₀-Kohlenwasserstoffrest ist, wobei M definiert ist als

      M = R⁸R⁹R¹⁰SiO_{1/2}

      worin R⁸, R⁹ und R¹⁰ jeweils unabhängig ein einwertiger C₁₋₄₀-Kohlenwasserstoffrest ist und
   (2) einem Harz der Formel:

      (M^{H}_{w}Q_{z})ⱼ

      worin Q die Formel SiO_{4/2} hat und M^{H} definiert ist als:

      H_{b}R¹¹_{3-b}SiO_{1/2}

      worin R¹¹ ein einwertiger C₁₋₄₀-Kohlenwasserstoffrest ist, wobei b eine Zahl von 1 bis 3 ist und w und z ein Verhältnis von 0,5 bis 4,0 haben und j im Bereich von 2,0 bis 100 liegt, wobei die Hydrosilylierung in Gegenwart von
   (3) Decamethylcyclopentasiloxan durchgeführt wird.
   Zwar kennt der Stand der Technik die EP 0827983, welche die erfindungsgemäßen Siloxanelastomere beschreibt, doch konnte diese Schrift nicht den Weg zur vorliegenden Erfindung weisen.
   Erfindungsgemäß besonders bevorzugt ist dieses Siloxanelastomere sind dadurch gekennzeichnet, dass
   R¹ und R⁶ = (CH₂=CH) sowie
   R², R³, R⁴, R⁵, R⁷, R⁸, R⁹ und R¹⁰ = CH₃ darstellen.
   Das erfindungsgemäße Siloxanelastomere kann beispielsweise bei der Firma Momentive Performance Materials unter der Handelsbezeichnung Silsoft Silicone Gel erworben werden.
   Es ist erfindungsgemäß vorteilhaft, wenn dieses erfindungsgemäße Siloxanelastomere A) in einer Konzentration von 5 bis 25 Gew.-% und bevorzugt in einer Konzentration von 10 bis 15 Gew.-%, jeweils bezogen auf das Gesamtgewicht der Zusammensetzung, eingesetzt wird.
B) Siloxanelastomers, welches hergestellt wird durch die Reaktion
   (A) eines ≡Si-H enthaltenden Polysiloxans der Formel R₃SiO(R'₂SiO)ₐ(R"HSiO)_{b}SiR₃ und, optional eines ≡Si-H enthaltenden Polysiloxans der Formel HR₂SiO(R'₂SiO)_{c}SiR₂H oder der Formel HR₂SiO(R'₂SiO)ₐ(R"HSiO)_{b}SiR₂H,
      mit R, R' und R" = Alkylgruppen mit 1 bis 6 C-Atomen,
      a=0-250 , b=1-250, und c=0-250,
   (B) mit einem alpha, omega-Dien der Formel CH₂=CH(CH₂)ₓCH=CH₂ mit x=1-20, wobei die Reaktion ausgeführt wird in Gegenwart eines Platin-Katalysators und (C) eines Lösungsmittels gewählt aus der Gruppe bestehend aus (i) organischen Komponenten, (ii) Komponenten enthaltend ein Silikonatom und deren beliebigen Mischungen,
      wobei die Reaktion solange durchgeführt wird, bis ein Gel durch Quervernetzung entstanden ist durch die Addition von ≡Si-H an die Doppelbindungen des alpha,omega-Diens.
      Zwar kennt der Stand der Technik die US 5654362, welche die erfindungsgemäßen Siloxanelastomere beschreibt, doch konnte diese Schrift nicht den Weg zur vorliegenden Erfindung weisen.
      Es ist erfindungsgemäß vorteilhaft, wenn dieses erfindungsgemäße Siloxanelastomere B) in einer Konzentration von 5 bis 25 Gew.-% und bevorzugt in einer Konzentration von 10 bis 15 Gew.-%, jeweils bezogen auf das Gesamtgewicht der Zusammensetzung, eingesetzt wird.
   C) Erfindungsgemäß am meisten bevorzugt ist das Siloxanelastomer mit der folgenden Struktur: 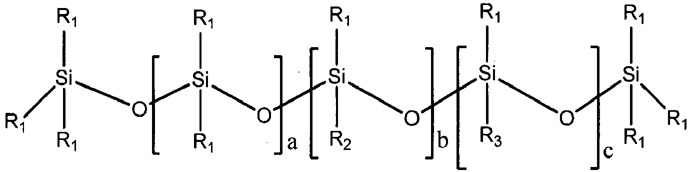 Mit
      R₁= C₁ bis C₃₀ Alkylrest, R₂=Wasserstoff,
      R₃= eine Quervernetzung -E-Y-E-, bei dem das andere Ende der Quervernetzung mit einer zweiten Silikonelastomerkette verbunden ist, jedes E eine bivalente Gruppe darstellt gewählt aus den Gruppen -CH₂-CH₂- oder -CH=CH- und Y eine bivalente Kohlenwasserstoff-Gruppe, ein Siloxan oder eine Kombination aus beidem darstellt,
      a = 265-2000, b= 0-249, C= 1-250, mit der Vorgabe, dass b+c ≤ 250,
      das in einem Lösungsmittel gequollen ist.
      Bei diesem Siloxanelastomer ist es erfindungsgemäß bevorzugt, wenn R₃ gewählt wird aus Polyethern, Pentylen, Hexylen, Heptylen, Octylen, Nonylen, Decylen, Dodecylen, Tetradecylen und Mischungen davon.
      Es ist bei diesem Siloxanelastomer erfindungsgemäß bevorzugt, wenn die Verbindung 0,01 bis 90 Mol-% Quervernetzer enthält.
      Bei diesem Siloxanelastomer ist es erfindungsgemäß bevorzugt, wenn als Lösungsmittel Decamethylcyclopentasiloxan eingesetzt wird.

Das erfindungsgemäße Siloxanelastomer C) lässt sich erfindungsgemäß vorteilhaft nach dem folgenden Verfahren herstellen:
1) Umsetzen in Gegenwart eines Hydrosilylierungskatalysators von
   (a) einem ≡Si-H haltigen Polysiloxan mit der Formel

      R¹₃SiO(R¹₂SiO)ₐ(R¹HSiO)_{d}SiR¹₃

      worin jedes R¹unabhängig voneinander eine monovalente Kohlenwasserstoffgruppe mit 1 bis 30 Kohlenstoffatomen ist, a gleich 265 bis 2000 ist und d gleich 1 bis 250 ist, mit
   (b) Dien-, Diin- oder En-In-Verbindung,
      wobei (a) und (b) in
   (c) einem Verdünnungsmittel dispergiert sind, und
2) Fortsetzen der Reaktion, bis ein Siliconelastomer durch Vernetzung und Addition von ≡Si-H an Doppelbindungen, in der Dien- Diin- oder En-In-Verbindung gebildet wird.

Dabei ist es erfindungsgemäß bevorzugt, wenn ein zusätzliches zweites ≡Si-H-haltiges Polysiloxan vorhanden ist, das ausgewählt wird aus HR¹₂SiO(R¹₂SiO)ₑSiR¹₂H, HR¹₂SiO(R¹₂SiO)ₐ(R¹HSiO)_{d}SiR¹₂H und Mischungen davon, worin jedes R¹ unabhängig voneinander eine monovalente Kohlenwasserstoffgruppe mit 1 bis 30 Kohlenstoffatomen ist, a gleich 265 bis 2000 ist und d gleich 1 bis 250 ist und e gleich 0 bis 250 ist, wobei das zweite ≡Si-H haltige Polysiloxan in einem Molverhältnis von >0 bis 20 vorhanden sind.

Erfindungsgemäß bevorzugt ist es ferner, wenn (b) ausgewählt wird aus 1,4-Pentadien, 1,5-Hexadien, 1,6-Heptadien, 1,7-Octadien, 1,8-Nonadien, 1,9-Decadien, 1,11-Dodecadien, 1,13-Tetradecadien und 1,19-Eicosadien, 1,3-Butadiin, 1,5-Hexadiin(Dipropargyl) und 1,Hexen-5-in.

Erfindungsgemäß bevorzugt ist es nicht zuletzt, wenn das Molverhältnis von (a) zu (b) im Bereich von 0,7:1 bis 1,3:1 liegt.

Diese erfindungsgemäßen Siloxanelastomere können beispielsweise bei der Firma Dow Corning unter der Handelsbezeichnung DC 9040, 9041 oder 9045 erworben werden.

Es ist erfindungsgemäß vorteilhaft, wenn dieses erfindungsgemäße Siloxanelastomere C) in einer Konzentration von 5 bis 25 Gew.-% und bevorzugt in einer Konzentration von 10 bis 15 Gew.-%, jeweils bezogen auf das Gesamtgewicht der Zusammensetzung, eingesetzt wird.

Es ist erfindungsgemäß vorteilhaft, wenn die erfindungsgemäße Zubereitung einen oder mehrere weitere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-borny-lidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethyl-hexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin; 2,4,6-Tribiphenyl-4-yl-1,3,5-triazin; Merocyanine; Titiandioxid; Zinkoxid.

Es ist im Rahmen der Erfindung sinnvoll, Wirkstoffe zur positiven Beeinflussung der Altershaut, die die Entstehung von Falten oder auch bestehenden Falten vermindern, einzusetzen. Als besonders bevorzugte Wirkstoffe gelten daher Biochinone, insbesondere Ubichinon Q10, Isoflavon und Isoflavonoide, Genistein, Arctiin, Cardiolipin, Liponsäure, Anti Freezing Proteine, Hopfen- und Hopfen-Malz-Extrakte und/oder die Restrukturierung des Bindegewebes fördernde Stoffe, Isoflavonoide sowie Isoflavonoid-haltige Pflanzenextrakte wie z.B. Soja- und Klee-Extrakte, die auch in den erfindungsgemäßen Zubereitungen und Hautauflagenmatrices sehr gut verwendet werden können. Auch zeigte sich, dass sich das erfindungsgemäße Kombinationsprodukt in besonderer Weise eignet, Wirkstoffe zur Unterstützung der Hautfunktionen bei trockener Haut, wie beispielsweise Vitamin C, Biotin, Kreatin, Kreatinin, Propionsäure, Glycerin, Grüntee-Extrakte, white tea - Extrakte oder - Lösungen, zu verwenden. In ähnlicher Weise erwies sich die Einarbeitung von Wirkstoffen zur Linderung bzw. positiven Beeinflussung von irritativen Hautzuständen, sei es bei empfindlicher Haut im Allgemeinen oder bei durch Noxen gereizter Haut (UV-Licht, Chemikalien), als vorteilhaft. Hier sind Wirkstoffe zu nennen wie Sericoside, verschiedene Extrakte des Süssholzes, Licochalcone A, Silymarin bzw. Silyphos, Flavonoide, Dexpanthenol, Ethanol, Inhibitoren des Prostaglandinstoffwechsels, insbesondere der Cyclooxygenase, und des Leukotrienstoffwechsels, insbesondere der 5-Lipoxygenase, aber auch des 5-Lipoxygenase Inhibitor Proteins, FLAP. Auch erwies sich die Einarbeitung von Modulatoren der Pigmentierung als vorteilhaft. Hier sind Wirkstoffe zu nennen, die die Pigmentierung der Haut vermindern und so zu einer kosmetisch gewünschten Aufhellung der Haut führen und/oder das Auftreten von Altersflecken reduzieren und/oder bestehende Altersflecken aufhellen, wie Tyrosinsulfat, Vitamin C, Liponsäure und Liponamid, verschiedene Extrakte des Süssholzes, Kojisäure, Hydrochinon, Arbutin, Fruchtsäuren, insbesondere Alpha-Hydroxy-Säuren (AHAs), Bearberry (Uvae ursi), Ursolsäure, Ascorbinsäure, Grüntee-Extrakte, Aminoguanidin und/oder Pyridoxamin. In gleicher Weise erwiesen sich die erfindungsgemäßen Nahrungsergänzungsmittel, Zubereitungen oder Matrices als hervorragende Grundlage für Wirkstoffe, die eine verstärkte/schnellere Bräunung der Haut herbeiführen (Advanced Glycation Endproducts (AGE), Lipofuscine, Nukleinsäure-Oligonukleotide, Purine und Pyrimidine, NO-freisetzende Substanzen, sei es mit oder ohne Einfluss von UV-Licht.

Für den Einsatz ebenfalls geeignete Wirkstoffe sind beispielsweise Folsäure, Phytoen, Harnstoff, D-Biotin, Coenzym Q10, Flavonglycoside wie z.B. α-Glucosylrutin, Carnitin, Polydocanol, natürliche und synthetische Isoflavonoide insbesondere Genistein, Flavonoide, Carotinoide, Kreatin, Kreatinin, Taurin, Ascorbinsäure sowie deren Derivate, Sauerstoff, Tocopherol sowie dessen Ester, Dihydroxyaceton, 8-Hexadecen-1,16-dicarbonsäure, langkettige Hyaluronsäuren (d.h. mit einem mittleren Molekulargewicht von 1 Million bis 3 Million Dalton) und kurzkettige Hyaluronsäuren (d.h. mit einem mittleren Molekulargewicht von 5000 bis 1 Million Dalton), Licochalcon A und deren Gemische. Derartige Inhaltsstoffe können jeweils in einer Einzelkonzentration von 0,01 bis 30 Gewichts-%, bezogen auf das Gesamtgewicht der Zubereitung, in dieser enthalten sein.

Erfindungsgemäße Formulierungen, welche ein oder mehrere Antifaltenwirkstoffe wie Flavonglycoside insbesondere α-Glycosylrutin, Coenzym Q10, Retinol und dessen Ester, Vitamin E und dessen Derivate sowie andere dem Fachmann bekannte Antifaltenwirkstoffe enthalten, eignen sich insbesondere zum Schutz vor ästhetisch unattraktiven Hautveränderungen wie sie beispielsweise bei ermüdeter Haut oder der Hautalterung auftreten. Zu diesen Veränderungen gehören z.B. Trockenheit, Rauhigkeit und Ausbildung von Trockenheitsfältchen, Juckreiz, verminderte Rückfettung vor allem nach dem Waschen, sichtbare Gefäßerweiterungen wie Teleangiektasien oder Cuperosis, Schlaffheit und Ausbildung von Falten und Fältchen, lokale Hyper-, Hypo- und Fehlpigmentierungen wie Altersflecken, vergrößerte Anfälligkeit gegenüber mechanischem Stress wie Rissigkeit und andere dem Fachmann bekannte Veränderungen. Weiterhin vorteilhaft eignen sich die erfindungsgemäßen Emulsionen gegen das Erscheinungsbild der trockenen bzw. rauhen Haut.

Weiterhin vorteilhaft ist der Einsatz von ein oder mehrere Antioxidantien in erfindungsgemäßen Rezepturen. Als geeignete Antioxidantien können ein oder mehrere Substanzen gewählt werden aus der Gruppe enthaltend Aminosäuren und deren Derivate wie z.B. Glycin, Histidin, Tyrosin und Tryptophan, Imidazole und deren Derivate wie z.B. Urocaninsäure, Peptide und deren Derivate wie z.B. D,L-Carnosin, D-Carnosin, L-Carnosin und Anserin, Carotinoide, Carotine und deren Derivate wie z.B. α-Carotin, β-Carotin und Lycopin, Liponsäure und deren Derivate wie z.B. Dihydroliponsäure, Aurothioglucose, Propylthiouracil und andere Thiole sowie deren Salze wie z.B. Thioredoxin, Glutathion, Cystein, Cystin, Cystamin sowie deren Glycosyl-, N-Acetyl-, Methyl-, Ethyl-, Propyl-, Amyl-, Butyl-, Lauryl-, Palmitoyl-, Oleyl-, γ-Linoleyl-, Cholesteryl - und Glycerylester, Dilaurylthiodipropionat, Distearylthiodipropionat, Thiodipropionsäure und deren Derivate wie Ester, Ether, Peptide, Lipide, Nukleotide, Nukleoside und Salze, Sulfoximinverbindungen wie z.B. Buthioninsulfoximine, Homocysteinsulfoximin, Buthioninsulfone, Penta-, Hexa- und Heptathioninsulfoximin in sehr geringen verträglichen Dosierungen (z.B. pmol bis µmol/kg), ferner (Metall)-Chelatoren wie z.B. α-Hydroxyfettsäuren, Palmitinsäure, Phytinsäure und Lactoferrin, α-Hydroxysäuren wie z.B. Zitronensäure, Milchsäure und Apfelsäure, Huminsäure, Gallensäure, Gallenextrakte, Bilirubin, Biliverdin, EDTA, EGTA und deren Derivate, ungesättigte Fettsäuren und deren Derivate wie z.B. γ-Linolensäure, Linolsäure und Ölsäure, Folsäure und deren Derivate, Ubichinon und Ubichinol und deren Derivate, Vitamin C und dessen Derivate wie z.B. Ascorbylpalmitat, Mg-Ascorbylphosphat und Ascorbylacetat, Tocopherole und deren Derivate wie z.B. Vitamin E-Acetat, Vitamin A und dessen Derivate wie z.B. Vitamin A-Palmitat, Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate wie z.B. ZnO und ZnSO₄, Selen und dessen Derivate wie z.B. Selenmethionin, Stilbene und deren Derivate wie z.B. Stilbenoxid und trans-Stilbenoxid, sowie die erfindungsgemäß geeigneten Derivate der genannten Wirkstoffe wie Salze, Ester, Ether, Zucker, Nukleotide, Nukleoside, Peptide oder Lipide.

Die Menge der Antioxidantien (eine oder mehrere Verbindungen) in den erfindungsgemäßen Formulierunge beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung. Sofern Vitamin E oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Zubereitung, zu wählen. Sofern Vitamin A, Vitamin-A-Derivate, Carotine oder deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Zubereitung, zu wählen. Es ist insbesondere vorteilhaft, wenn die kosmetischen Emulsionen gemäß der vorliegenden Erfindung kosmetische Wirkstoffe enthalten, wobei bevorzugte Wirkstoffe Antioxidantien sind, welche die Haut vor oxidativer Beanspruchung schützen können.

Es ist weiterhin im Rahmen der Erfindung vorteilhaft, wenn Substanzen zur verbesserung der mikrobioligischen Stabilität, insbesondere Substanzen mit antimikrobieller Wirkung, insbesonderer gegen Bakterien, Hefen und Pilze, eingesetzt werden. Als bevorzugt zu nennen sind hierbei Benzoesäure, ihre Ester und Salze, Propionsäure und ihre Salze, Salicylsäure und ihre Salze, 2,4-Hexadiensäure (Sorbinsäure) und ihre Salze, Formaldehyd und Paraformaldehyd, 2-Hydroxydiphenyl, Zinkpyrithion, Anorganische Sulfite und Bisulfite, Chlorobutanolum, 4-Hydroxybenzoesäure, ihre Salze und Ester, 3-Acetyl-6-methyl-2,4(3H)-pyrandion (Dehydracetsäure) und seine Salze, Ameisensäure und ihr Natriumsalz, 1,6-Bis(4-amidino- 2-bromphenoxy)-n-hexan (Dibromhexamidin) und seine Salze, Ethylquecksilber-(II)-thiosalicylsäure, Natriumsalz (Thiomersalum), Phenylquecksilber und seine Salze, 10-Undecylensäure und ihre Salze, 5-Amino-1,3-bis(2-ethylhexyl)-5-methyl-hexahydropyrimidin (Hexetidinum), 5-Brom-5-nitro-1,3-dioxan, 2-Brom-2-nitro-1,3-propandiol (Bronopol), 2,4-Dichlorbenzylalkohol, N-(4-Chlorphenyl)-N'-(3,4-dichlorphenyl)-harnstoff (Triclocarban), 4-Chlorm-cresol, 2,4,4'-Trichlor-2'-hydroxydiphenylether (Triclosanum), 4-Chlor-3,5-dimethylphenol, 1,1'-Methylenbis(3-(1-hydroxymethyl-2,5-dioximidazolidin-4-yl)harnstoff) (Imidazolidinylharnstoff), Poly(hexamethyl-endiguanid)hydrochlorid, 2-Phenoxy-ethanol, Hexamethylentetramin, 1-(3-Chlorallyl)-3,5,7-triaza-1-azonia-adamantanchlorid, 1-(4-Chlorphenoxy)-1-(imidazol-1-yl)-3,3-dimethyl-2-butanon, 1,3-Bis-(hydroxymethyl)-5,5-dimethyl-2,4-imidazolidindion, Benzylalkohol, 1-Hydroxy-4-methyl-6-(2,4,4-trimethylpentyl)-2-pyridon und sein Monoethanolaminsalz, 2,2'-Methylen-bis(6-brom-4-chlorphenol) (Bromchlorophen), 3-Methyl-4-(1-methylethyl)phenol, Mischung von 5-Chlor-2-methyl-3(2H)-isothiazolon und 2-methyl-3(2H)-isothiazolon mit Magnesiumchlorid und Magnesiumnitrat, 2-Benzyl-4-chlorphenol (Chlorophenum), 2-Chloracetamid, Chlorhexidin, sein Acetat, Gluconat und Hydrochlorid, 1-Phenoxy-propan-2-ol, N-Alkyl (C12-C22)trimethylammoniumbromid und -chlorid, 4,4-Dimethyl-1,3-oxazolidin, N-Hydroxymethyl-N-(1,3-di(hydroxymethyl)-2,5-dioxoimidazolidin-4-yl)-N'-hydroxymethyl-harnstoff, 1,6-Bis(4-amidinophenoxy)-n-hexan (Hexamidinum) und seine Salze, Glutaraldehyd (Pentan-1,5-dial), 5-Ethyl-1-aza-3,7-dioxabicyclo(3.3.0)octan, 3-(4-Chlorphenoxy)-1,2-propandiol (Chlorphenesin), Natriumhydroxymethylaminoacetat, Silberchlorid, Benzethoniumchlorid, Benzalkoniumchlorid, -bromid und -saccharinat, Benzylhemiformal, lodopropinylbutylcarbamat (IPBC), 3-lod-2-propinylbutylcarbamat, 2-Methyl-3-(2H)-isothiazolon (Methylisothiazolinone), Piroctone Olamine und Ethyl lauroyl arginate. Insbesondere vorteilhaft sind Methylisothiazolinone, die Kombinationen von Ethyl lauryl arginate mit Phenoxyethanol, Benzethoniumchlorid mit Phenoxyethanol oder Piroctone Olamine mit Phenoxyethanol.

Zudem können die erfindungsgemäßen Formulierungen weitere, in kosmetischen Formulierungen bekannte Inhaltsstoffe enthalten, wie z.B. Acetyl Trifluoromethylphenyl Valylglycine, Acrylamide / Ammonium Acrylate Copolymer, Acrylates/C12-22 Alkylmethacrylate Copolymer, Aluminum / Magnesium Hydroxide Stearate, Ammonium Lactate, Ammonium Polyacrylate, Ammonium Polyacryloyldimethyl Taurate, Arginine PCA, Beerwax, Benzethoniumchlorid, Capryloyl Salicylic Acid, β-Carotin, Cinnamic Acid, Coco Glucoside, Copper Gluconate, Dehydroxanthan Gum, Diphenyl Dimethicone, Disodium Adenosine Triphosphate, Disodium Succinate, Disteardimonium Hectorite, Dodecene, Eperua Falcata, Hydrogenated Palm Glycerides Citrate, Hydrogenated Palm Kernel Glycerides, Hydrolyzed Wheat Protein, PG-Propyl Methylsilanediol, Kasei, Kinetin, Hydroxyethyl Acrylate / Sodium Acryloyldimethyltaurate Copolymer, Isodeceth-6, Linseed Acid, Lutein, Lyopene, Magnesium Aspartate, Melibiose, Oxothiazolidinecarboxylic Acid, Palmitoyl Pentapeptide 4, PEG-8 Laurate, PG-10 Stearat, Phenethyl Alcohol, Phenylpropanol, Polyacrylate-13, Polyacrylate-3, Quinic Acid, Sarcosine, Saxifraga Sarmentosa Extract, Scutellaria Baicalensis Extract, Shikimic Acid, Sodium Metabisulfite, Soy Isoflavone, Tocopheryl Glucoside, Trideceth-6, Zeaxanthin, Zinc Gluconate, Triacetin, 1,2 Hexandiol, Hydroxyethylpiperazine Ethane Sulfonic Acid, Nicotinamide, Penethyl Alcohol, Penthylene Glycol, Carnaubau Wax, Chlorhexidine Digluconate, Oleyl Erucate, Acetyltrifluoromethylphenylvalylglycin, Acrylamidammoniumacrylatcopolymer, Aluminummagnesiumhydroxidstearat, Ammoniumlactat, Ammoniumpolyacrylat, Ammoniumpolyacryloyldimethyltaurat, Arginin PCA, Capryloylsalicylsäureester, Zimtsäure, Cocoglucosid, Kupfergluconat, Diphenyldimethicon, Dinatriumadenosintriphosphat, Dnatriumsuccinat, Disteardimoniumhectorit, Dodecen, Eperua Falcata , hydriertes Palmenglycerid, hydriertes Palmenglyceridcitrat, Hydrierte Palmenkernglyceride, Hydrolisiertes Weizenprotein PG-Propylmethylsilandiol, Hydroxyethylacrylat / Natriumacryloyldimethyltauracopolymer, Isodeceth-6, Linseed Acid, Magnesiumaspartat, Melibiose, Oxothiazolidinecarboxylsäure, Palmitoylpentapeptide 4, PEG-8 Laurat, Phenethylalkohol, Phenylpropanol, Polyacrylate-13, Polyacrylate-3 , Sarcosin, Saxifraga Sarmentosa Extrakt, Scutellaria Baicalensis Extrakt, Natriummetabisulfit, Sojaisoflavon, Tocopherylglucosid, Trideceth-6, Zinkgluconat, Triacetin, 1,2 Hexandiol, Hydroxyethylpiperazine Ethane Sulfonic Acid, Nicotinamide, Penethyl Alcohol, Penthylene Glycol, Carnaubau Wax, Chlorhexidine Digluconate, Oleyl Erucate.

Zudem ist die Verwendung von Stoffen zur positiven Beeinflussung des Geruchs der Formuliuerung sinnvoll. Hierzu zählen z.B. Dipropylene glycol, Methyl dihydrojasmonate, Phenethyl alcohol, Linalool, Linalyl acetate, 2,6-Dimethyl-7-octen-2-ol, alpha-Hexylcinnamaldehyde, 2-Acetonapthone-1,2,3,4,5,6,7,8-octahydro-2,3,8,8-tetramethyl p-t-Butylalpha-methyldihydrocinnamic aldehyde, Benzyl acetate, 1,3,4,6,7,8-Hexahydro-4,6,6,7,8,8-hexamethylcyclopenta-gamma-2-benzopyran, Methyl cedryl ketone, Ethylene brassylate, 4-(4-Hydroxy-4-methylpentyl)-3-cyclohexene-1-carboxyaldehyde, Benzyl salicylate, Hexyl salicylate Orange oil, alpha-Isomethylionone, Diethyl phthalate, 4-t-Butylcyclohexyl acetate, Patchouli oil 3,7-Dimethyl-2,6-octadien-1-ol, Tetrahydrolinalool, Hydroxycitronellal, Isopropyl myristate, 3,7-Dimethyl-6-octen-1-ol, Orange terpenes, Heliotropin, Terpinyl acetate, omega-Pentadecalactone, Methyl-alpha-ionone, Lavandin oil, Lemon oil, Bergamot oil, 7-Acetyl-1,1,3,4,4,6-hexamethyltetralin, Coumarin, Ethyllinalool, Amyl salicylate, 2-tert-Pentyl-cyclohexyl acetate, 3-Methyl-5-phenyl-1-pentanol, Cedrol, Benzyl benzoate, Vanillin, alpha-Amylcinnamaldehyde, Dimethyl phthalate, d-Limonene, 2-Isobutyl-4-hydroxy-4-methyltetrahydropyran, Triethyl citrate, Terpineol, Lavender oil, Diethylene glycol monoethyl ether, 2-Phenoxyethyl isobutyrate, Anisyl alcohol, 3-Pentyltetrahydro(2H)pyranyl acetate, Methyl ester of rosin, partially hydrogenated, Isobornyl acetate, Rosemary oil, Petitgrain oil, 1,4-Dioxacyclohexadecane-5,16-dione, Isoamyl salicylate, gamma-Undecalactone, alpha-Ionone, Oxacyclohexadecen-2-one, 7-Octen-2-ol, 2-methyl-6-methylene, dihydro derive. 1,2-Propylene glycol, 3-(5,5,6-Trimethylbicyclo(2.2.1)hept-2-yl)cyclohexan-1-ol, Geranium oil, Musk ketone, Cedrenyl acetate, Isobornylcyclohexanol, lonone, Benzyl alcohol, gamma-Nonalactone, I-Menthol, Cyclohexyl salicylate, Dihydromyrcenyl acetate, Citral, Orange terpenes (natural), Cedarwood oil, alpha-Pinene, Majantol, Phenoxyethanol, Ethyl acetate, Cedrol methyl ether, 1,5,9-Trimethyl-13-oxabicyclo(10.1.0) trideca-4,8-diene, Peppermint oil, Eugenol, Ethyl maltol, Benzaldehyde, Cinnamic alcohol, 3,7-Dimethyl-1-octanol, alpha-Methyl-3,4-methylene dioxyhydrocinnamic aldehyde, beta-Pinene, d-Camphor, Methyl abietate, Cedryl acetate, Ylang ylang oil, Sandalwood oil, Mineral oil, Dimethyl benzyl carbinyl butyrate, Ethyl butyrate, Geranyl acetate, Hexylene glycol, Myrcene, alpha-Methyionantheme, beta-Ionone, 3-(4-t-Butylphenyl)propanal, 3,7-Dimethyloctan-3-yl acetate, Acetic acid, (1-oxopropoxy)-,1-(3,3-dimethylcyclohexyl), Eucalyptol, 4-Carvomenthenol, Stearic acid, Menthanyl acetate, Eucalyptus oil, Dihydroterpinyl acetate, o-t-Butylcyclohexyl acetate, Isoeugenol, alpha-Terpineol, Cyclamen aldehyde, Hydroxycitronellol, Myrcenyl acetate, Nopyl acetate, 3,7-Dimethyl-1,3,6-octatriene, Rhodinol, Dimethyl benzyl carbinyl acetate, Tricyclodecenyl propionate, 2-Methyl-5-phenylpentan-1-ol, Sclareoate, 3-Isocamphyl cyclohexanol, trans-Anethole, Hexahydro-4,7-methanoinden-5(6)-yl acetate, 4-(p-Hydroxyphenyl)-2-butanone, Nerolidol, alpha-Butylcinnamaldehyde, Bornyl acetate, Etyhl methylphenylglycidate, trans-betalonone, Camphene, Juniper berry oil, Mandarin oil, Nutmeg oil, Spearmint oil, Grapefruit oil, Labdanum oil, Galbanum oil, Menthone, Trichloromethyl phenyl carbinyl acetate, alpha-Methylbenzyl acetate, Ethyl-2methyl-1,3-dioxolane-2-acetate, 2,6-Nonadienal, Abietyl acetate, Anisic acid, Diphenyl ether, Triacetin, 2-Methyl-4-phenyl-2-butanol Phenylethyl acetate, 1-Phenyl-3-methyl-3-pentanol, Anisyl acetate, Cinnamic aldehyde, p-Methylanisole, 5-Phenylpentanol, Diethyl malonate, Citronellal, Nerol, Undecanal, 2-methyl-, Hexyl alcohol, Glyceryl caprylate, Methyl 2-nonenoate, Octyl acetate, Decanal, Lauryl alcohol, Lauric aldehyde, Ethyl vanillin, 3-Phenyl-1-propanol, Octanal, Butylated hydroxytoluene, 4-Acetyl-6-t-butyl-1,1-dimetylindane, delta-3-Caren, Benzyl laurate, Neryl acetate, Ethyl acetoacetate, Hexyl acetate, Menthol liquid, Citronellyl acetate, Tetrahydromyrcenol, Diacetin, Menthyl acetate, 3(4),8(9)-Dihydroxymethyl tricyclo(5.2.1.0(2,6)decane, 2,4-Dimethyl-3-cyclohexen-1-carboxaaldehyde, Cedrenol, Phenylacetaldehyde glyceryl acetal, Sabinene, 3,7,11-Trimethyl-1,2,10,-dodecatrien-3-ol (cis & trans), Octyldodecanol, Formaldehyde cyclododecyl ethyl acetal Myristicin, 3,7-Dimethyl-2(3),6-nonadienenitrile, Ethyllinylyl acetate, 2-Methylbutyl acetate, cis-3-Hexenyl salicylate, 2-Methyl-4-(2,6,6-trimethyl-2(1)-cyclohexen-1-yl) butanal, Maltol isobutyrate, 2-Methyl-3(4-(2-methylpropyl)phenyl)propanal, 12-Oxahexadecanolide, 1,1-Dimethoxy-2,25-trimethyl-4-hexene, 1,6,7,8-Tetrahydro-1,4,6,6,8,8-hexamthyl-as-indacen-3<2H>-one, Bergamot oil, bergaptene free, Treemoss abs., Citrus oil distilled, Lemon terpenes, gamma-Decalactone, 2-Methyl-4-phenyl-2-pentanone, Allyl phenoxyacetate, Methyl-delta-ionone, Citronella oil, Clove bud oil, Thyme oil, Lime oil, Bois de rose oil, Cognac oil, Neroli bigarade oil, Spike lavender oil, Vetiver oil, Fir needle oil, Methylpentenolone, Lemon oil terpenes, Isobutyl salicylate, beta-Caryophyllene, Pulegone, Thymol, gamma-Terpinene.

### Vergleichsversuch/Ausführungsbeispiel

Es wurden die in Tabelle 1 beschriebenen O/W Emulsionen zur Hautpflegehergestellt. Die Rezeptur #1 ist eine erfindungsgemäße Ausführungsform; die Beispiele C1 und C2 sind nicht erfindungsgemäße Vergleichsrezepturen. Alle Mengenangaben beziehen sich auf Gewichts-%.

| Inhaltsstoff | Bsp. #1 | Bsp. C1 | Bsp. C2 |
|---|---|---|---|
| Glyceryl Stearate Citrate | 2,5 | 2,5 | 2,5 |
| Octocrylene | 2 | 2 | 2 |
| Ethylhexyl Salicylate | 4 | 4 | 4 |
| Butyl Methoxydibenzoylmethane | 4 | 4 | 4 |
| Butylene Glycol Dicaprylate/Dicaprate | 2 | 2 | 2 |
| Cyclomethicone + Cetearyl Dimethicone/Vinyl Dimethicone Crosspolymer¹⁾ | 15 | 15 | 15 |
| Tapioca Starch + Aqua | 2 | | |
| Dimethicone/Vinyl Dimethicone Crosspolymer + Silica ²⁾ | | 2 | |
| Glycerin | 10 | 10 | 10 |
| Sodium Carbomer | 0,6 | 0,6 | 0,6 |
| Alcohol Denat. | 5 | 5 | 5 |
| Aqua | ad 100 | ad 100 | ad 100 |
| Actives | eq. | eq. | eq. |
| Preservative | eq. | eq. | eq. |
| Parfum | eq. | eq. | eq. |
| ¹⁾ Silsoft Silicone Gel (Momentive) - erfindungsgemäßes Silikonelastomergel | | | |
| ²⁾ 9701 Cosmetic Powder (Dow Corninig) - nicht erfindungsgemäße Silikonelastomerpartikel | | | |

### Ermittelte Wasserfestigkeiten durch Kontaktwinkelmessung:

Bei 8 Probanden werden auf der gereinigten Unterarminnenseite pro Testprodukt 1,5 mg/cm² Formulierung auf einer 4x6 cm großen Fläche appliziert. Nach 20 Minuten wird ein Wassertropfen von 8 µl aufgetropft. Dieser Tropfen wird nun betrachtet und mit einer CCD-Kamera aufgenommen. An dem in den Computer eingelesenen Bild wird eine Konturanalyse vorgenommen und durch Anlegen einer Tangente der Kontaktwinkel bestimmt. Ein hoher Kontaktwinkel steht dabei für eine hohe Wasserfestigkeit.
Bsp.1: 62°
Bsp. C1: 57°
Bsp. C2: 53°

Die Verwendung von Tapioca Stärke (Bsp. 1) führt zu einem signifikanten Anstieg des Kontaktwinkels und deutet so auf eine gesteigerte Wasserfestigkeit hin.

### Viskositäten

Von allen drei Rezepturen wurde mit Hilfe eines Rotationsrheometers mit der Bezeichnung Rheomat R 123 der Firma proRheo GmbH mit dem Messkörper 1 bei einer Temperatur von 25°C bestimmt.
Bsp. 1: >10000 mPas
Bsp. C1: 8500 mPas
Bsp. C2: 2800 mPas

Beide Puderrohstoffe führen zu einer Erhöhung der Viskosität, wobei die erfindungsgemäße Formulierung hier noch deutlich effektiver ist.

### Sensorische Evaluierung (Panel-Bewertung)

10 geschulte Panelisten bewerten nach festgelegten sensorischen Attributen die Formulierungen. Dazu werden auf kreisrunden Arealen auf den Unterarminnenseiten 50 µl Produkt aufgetragen und in kreisenden Bewegungen (Takt von 1 Umdrehung pro Sekunde) verteilt. Es folgt eine Bewertung der sensorischen Attribute nach 20 Kreisen, nach vollständigem Einziehen sowie 5 Minunten nach dem Verteilen. Die Bewertung erfolgt auf einer Skala von 1 bis 10. Ein Unterschied von etwa 1 Punkt bedeutet nach den Erfahrungen ein vom ungeschulten Verbraucher merkbarer Unterschied.

Parameter "Frisch/wässrig beim Verteilen"
Bsp.1: 6,5
Bsp. C1: 5,0
Bsp. C2: 6,6

Die erfindungsgemäße Formulierung fühlt sich beim Verteilen genau so leicht und frisch an wie eine Formulierung ohne Puderrohstoff (C2).

Parameter "öliger Rückstand nach dem Verteilen"
Bsp. 1: 2,2
Bsp. C1: 2,5
Bsp. C2: 3,2

Die erfindungsgemäße Formulierung zeigt eine bessere Maskierung der Öle als die anderen Formulierungen (C1 und C2).

Parameter "wachsig / stumpfes Hautgefühl 5 Minuten nach Verteilen"
Bsp. 1: 1,6
Bsp. C1: 1,7
Bsp. C2: 2,5

Die Formulierungen mit Puderrohstoffen (Bsp. 1 und C1) haben ein glatteres Hautgefühl als die Formulierung ohne Puderrohstoff (C2).

Lediglich die erfindungsgemäße Formulierung zeigt eine leichte, frische Sensorik beim Verteilen, geringe ölige Rückstände auf der Haut und ein glattes Hautgefühl.

Weitere erfindungsgemäße Formulierungen sind z.B.

| Inhaltsstoff | Bsp. #2 | Bsp. #3 | Bsp. #4 | Bsp. #5 | Bsp. #6 |
|---|---|---|---|---|---|
| Glyceryl Stearate Citrate | | | | 2 | 2 |
| Potassium Cetyl Phosphate | 0,4 | | | | |
| Polyglyceryl-3 Stearate | | 2 | | | |
| Sodium Stearoyl Glutamate | | | 0,2 | | |
| Cetearyl Alcohol | 1 | 1 | 1 | 1 | 1 |
| Octocrylene | 2 | 2 | 2 | 2 | 2 |
| Ethylhexyl Salicylate | 4 | 4 | 4 | 4 | 4 |
| Butyl Methoxydibenzoylmethane | 4 | 2 | 2 | 4 | 4 |
| Phenylbenzimidazole Sulfonic Acid | | 1 | 1 | | 1 |
| Butylene Glycol Dicaprylate/Dicaprate | 2 | 2 | 2 | 2 | 2 |
| Cyclomethicone + Cetearyl Dimethicone/Vinyl Dimethicone Crosspolymer ¹⁾ | 15 | 15 | 15 | | |
| Cyclomethicone + Dimethicone Crosspolymer ²⁾ | | | | 15 | 15 |
| Tapioca Starch + Aqua | 2 | 2 | 2 | 2 | 2 |
| Dimethicone/Vinyl Dimethicone Crosspolymer + Silica ³⁾ | | | | | |
| Glycerin | 10 | 10 | 10 | 10 | 10 |
| Sodium Carbomer | | | | | |
| Carbomer | 0,5 | 0,5 | 0,5 | 0,5 | 0,5 |
| Sodium Hydroxide | eq. | eq. | eq. | eq. | eq. |
| Alcohol Denat. | 5 | 5 | 5 | | |
| Aqua | ad 100 | ad 100 | ad 100 | ad 100 | ad 100 |
| Actives | eq. | eq. | eq. | eq. | eq. |
| Preservative | eq. | eq. | eq. | eq. | eq. |
| Parfum | eq. | eq. | eq. | eq. | eq. |
| ¹⁾ Silsoft Silicone Gel (Momentive) - erfindungsgemäßes Silikonelastomergel | | | | | |
| ²⁾ 9040 Silicone Elastomer Blend (Dow Corning) - erfindungsgemäßes Silikonelastomergel | | | | | |
| ³⁾ 9701 Cosmetic Powder (Dow Corninig) - nicht erfindungsgemäße Silikonelastomerpartikel | | | | | |

## Patentansprüche

1. Kosmetische Zubereitung in Form einer O/W-Emulsion enthaltend
a) ein oder mehrere UV-Filter
b) ein oder mehrere Silikoneiastomergele,
die Zubereitung mindestens 10 Gew.-%. bezogen auf das Gesamtgewicht der Zubereitung, an Silikonelastomergel enthält,
c) mindestens 1,5 Gew.-%, bezogen auf das Gesamtgewicht der Zubereitung, an Tapiokastärke,
**dadurch gekennzeichnet, dass** die Zubereitung 4-(tert,-Butyl)-4'-methCxydibenzoylmethan enthält.

2. Kosmetische Zubereitung nach Anspruch 1, **dadurch gekennzeichnet, dass** die Zubereitung mindestens 5 Gew.-%, bezogen auf das Gesamtgewicht der Zubereitung, an UV-Filtern enthält.

3. Kosmetische Zubereitung nach einem der vorhergehenden Ansprüche, **dadurch gekennzeichnet, dass** die Zubereitung einen oder mehrere weitere UV-Filter enthält, gewählt aus der Gruppe der Verbindungen 2-Phenylbenzimidazol-5-sulfonsäure und/oder deren Salze; Phenylen-1,4-bis-(2-banzimidazyl)-3,3'-5,5'-tetrasuifonsäuresaize; 1,4-di(2-oxo-10-Sulfo-3-bornylidenmethyl)-Benzol und dessen Salze; 4-(2-Oxo-3-bornylidenmethyl)benzolsulfonsäuresafze; 2-Methyl-5-(2-oxo-3-bornylidenmethyl)sulfonsäuresalze; 2,2'-Methylen-bis-(6-(2H-benzotriazol-2-y))-4-(1,1,3,3-tetramethylbutyl)-phenol); 2-(2H-benzotriazol-2-yl)-4-methyl-6-[2-methyl-3-[1,3,3,3-tetramethyl-1-[(trimethylsily)oxy]disiloxanyl]propyl]-phenol; 3-(4-Methytbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethythexyl)ester; 4-(Dimethylamino)benzoesäureamylester; 4-Methoxybenzalmalon-säuredi(2-ethylhexyl)ester; 4-Meihoxyzimtsäure(2-ethylhexyl)ester; 4-Methoxyzimtsäureisoemylester; 2-Hydroxy-4-methoxybenzophenon, 2-Hydroxy-4-methoxy-4'-methylbenzophetion; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmatonat; 3-(4-(2,2-bis Ethoxycarbonylvinyl)-phenoxy)propenyl)-methoxysiloxan / Dimethylsiloxan-Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4.4'.4"-(1,3,5-Triazin-2.4,6.triyltrimino)-tris-benzoësäure-tris-(2-ethylhexylester) (auch: 2,4,6-Tris-[anilino-(p-carbo-2'-ethyl-1'-hexyloxy)]-1,3,5-triazin (INCI: Ethylhexyl Triazone); 2.4-Bis-[4-(2-ethyl-hexyloxy)2-hydroxy]-phenyl)-6-(4-methoxyphenyl)-1.3,5-triazin; 2,4,6-Tribiphenyl-4-yl-1,3,5-triaziri; Merocyanine; Titiandioxid; Zinkoxid.

4. Kosmetische Zubereitung nach einem der vorhergehenden Ansprüche, **dadurch gekennzeichnet, dass** das Siloxanelastomer hergestellt wird durch das Hydrosilylierungs-Produkt von
(1) einem linearen Alkenyl-Endgruppen aufweisenden Polyorganosiloxan der Formel:
M^{vi}ₛDₓD^{vi}_{y}M₂₋ₐ
worin x eine Zahl von 450 bis 1250, y eine Zahl von 0 bis 200, a eine Zahl von 0 bis 2 ist, unter der Einschränkung dass a+y im Bereich von 1 bis 20 liegt, wobei M^{vi} definiert ist als
R¹R²R³SiO_{1/2}
worin R¹ ein einwertiger ungesättigter Kohlenwasserstoffrest mit 2 Kohlenstoffatomen ist und R² und R³ jeweils unabhängig einwertige C₁ bis C₄₀-Kohlenwasserstoffreste sind, wobei D definiert ist als
R⁴R⁵SiO_{2/2}
worin R⁴ und R³ jeweils unabhängig einwertige C₁₋₄₀-Kohllenstoffreste sind, wobei D^{vi} definiert ist als
D^{vi}=R⁶R⁷SiO_{2/2}
worin R⁶ ein einwertiger ungesättigter Kohlenwasserstoffrest mit 2 bis 10 Kohlenstoffatomen ist und R⁷ unabhängig ein einwertiger C₁₋₄₀-Kohlenwasserstoffrest ist, wobei M definiert ist als
M = R⁸R⁹R¹⁰SiO_{1/2}
worin R⁸, R⁹ und R¹⁰ jeweils unabhängig ein einwertiger C₁₋₄₀-Kohlenwasserstofirest ist
und
(2) einem Harz der Formel:
(M^{H}_{w}O_{z})ⱼ
worin Q die Formel SiO_{4/2} hat und M^{H} definiert ist als:
H_{b}R¹¹_{3-b}SiO_{1/2}
worin R¹¹ ein einwertiger C₁₋₄₀-Kohlenwasserstoffrest ist, wobel b eine Zahl von 1 bis 3 ist und w und z ein Verhältnis von 0,5 bis 4,0 haben und j im Bereich von 2,0 bis 100 liegt,
wobei die Hydrosilylierung in Gegenwart von
(3) Decamethylcyclopentasiloxan durchgeführt wird.

5. Kosmetische Zubereitung nach Anspruch 6 **dadurch gekennzeichnet, dass**
R¹ und R⁶ = (CH₂=CH) sowie
R², R³, R⁴, R⁵, R⁷, R⁸, R⁹ und R¹⁰ = CH₃ darstellen.

6. Kosmetische Zubereitung nach einem der Ansprüche 1 bis 5, **dadurch gekennzeichnet, dass** das Siloxanelastomer die folgende Struktur aufweist: 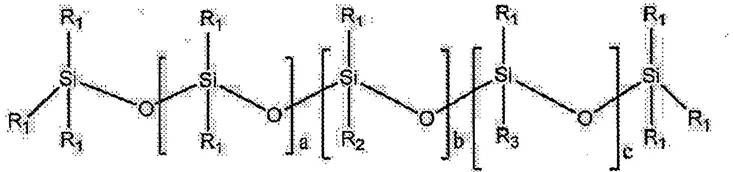 mit
R₁= C₁ bis C₃₀ Alkylrest, R₂=Wasserstoff,
R₃= eine Quervernetzung -E-Y-E-, bei dem das andere Ende der Quervernetzung mit einer zweiten Silikonelastomerkette verbunden ist, jedes E eine bivalente Gruppe darstellt gewählt aus den Gruppen -CH₂-CH₂- oder -CH=CH- und Y eine bivalente Kohlenwasserstoff-Gruppe, ein Siloxan oder eine Kombination aus beidem darstellt, a = 265-2000, b= 0-249, C= 1-2-50, mit der Vorgabe, dass b+c ≤250,
das in einem Lösungsmittel gequollen ist.

7. Kosmetische Zubereitung nach Anspruch 8, **dadurch gekennzeichnet, dass** bei dem Siloxanelastomer R₃ gewählt wird aus Polyethern, Pentylen, Mexylen, Heptylen, Octylen, Nonylen, Decylen, Dodecylen, Tetradecylen und Mischungen davon.

## Claims

1. Cosmetic preparation in the form of an O/W emulsion comprising
a) one or more UV filters
b) one or more silicone elastomer gels,
the preparation comprises at least 10% by weight of silicone elastomer gel, based on the total weight of the preparation,
c) at least 1.5% by weight of tapioca starch, based on the total weight of the preparation,
**characterized in that** the preparation comprises 4-(tert-butyl)-4'-methoxydibenzoylmethane.

2. Cosmetic preparation according to Claim 1, **characterized in that** the preparation comprises at least 5% by weight of UV filters, based on the total weight of the preparation.

3. Cosmetic preparation according to either of the preceding claims, **characterized in that** the preparation comprises one or more further UV filters selected from the group of compounds comprising 2-phenylbenzimidazole-5-sulphonic acid and/or salts thereof; phenylene-1,4-bis(2-benzimidazyl)-3,3'-5,5'-tetrasulphonic acid salts; 1,4-di(2-oxo-10-sulpho-3-bornylidenemethyl)benzene and salts thereof; 4-(2-oxo-3-bornylidenemethyl)benzenesulphonic acid salts; 2-methyl-5-(2-oxo-3-bornylidenemethyl)sulphonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulphonic acid; 2-ethylhexyl 2-cyano-3,3-diphenylacrylate; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; hexyl 2-(4'-diethylamino-2'-hydroxybenzoyl)benzoate; homomenthyl salicylate; 2-ethylhexyl2-hydroxybenzoate; dimethicodiethylbenzalmalonate; 3-(4-(2,2-bis ethoxycarbonylvinyl)phenoxy)propenyl)methoxysiloxane / dimethylsiloxane - copolymer; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4-bis-{[4-(2-ethylhexyloxy)-2-hydroxy]phenyl}-6-(4-methoxyphenyl)-1,3,5-triazine; 2,4,6-tribiphenyl-4-yl-1,3,5-triazine; merocyanine; titanium dioxide; zinc oxide.

4. Cosmetic preparation according to any of the preceding claims, **characterized in that** the siloxane elastomer is prepared by the hydrosilylation product of
(1) a polyorganosiloxane having linear alkenyl end groups of the formula:
M^{vi}ₐDₓD^{vi}_{y}M₂₋ₐ
where x is a number from 450 to 1250, y is a number from 0 to 200, a is a number from 0 to 2, with the limitation that a+y is in the range of 1 to 20, wherein M^{vi} is defined as
R¹R²R³SiO_{1/2}
where R¹ is a monovalent unsaturated hydrocarbon radical having 2 carbon atoms and R² and R³ are each independently monovalent C₁ to C₄₀ hydrocarbon radicals, where D is defined as
R⁴R⁵SiO_{2/2}
where R⁴ and R⁵ are each independently monovalent C₁₋₄₀ carbon radicals, wherein D^{vi} is defined as
D^{vi}=R⁶R⁷SiO_{2/2}
where R⁶ is a monovalent unsaturated hydrocarbon radical having 2 to 10 carbon atoms and R⁷ is independently a monovalent C₁₋₄₀ hydrocarbon radical, wherein M is defined as
M = R⁸R⁹R¹⁰SiO_{1/2}
where R⁸, R⁹ and R¹⁰ are each independently a monovalent C₁₋₄₀ hydrocarbon radical
and
(2) a resin of the formula:
(M^{H}_{w}Q₂)ⱼ
where Q has the formula SiO_{4/2} and M^{H} is defined as:
H_{b}R¹¹_{3-b}SO_{1/2}
where R¹¹ is a monovalent C₁₋₄₀ hydrocarbon radical, where b is a number from 1 to 3 and w and z have a ratio of 0.5 to 4.0 and j is in the range of 2.0 to 100,
wherein the hydrosilylation is carried out in the presence of
(3) decamethylcyclopentasiloxane.

5. Cosmetic preparation according to Claim 6, **characterized in that**
R¹ and R⁶ = (CH₂=CH) and
R², R³, R⁴, R⁵, R⁷, R⁸, R⁹ and R¹⁰ = CH₃.

6. Cosmetic preparation according to any of Claims 1 to 5, **characterized in that** the siloxane elastomer has the following structure: 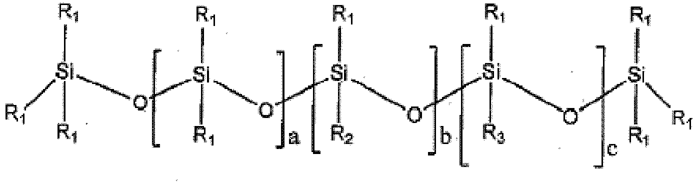 where
R¹ = C₁ to C₃₀ alkyl radical, R₂ = hydrogen,
R₃ = a crosslinking -E-Y-E-, in which the other end of the crosslinking is attached to a second silicone elastomer chain, each E is a bivalent group selected from the groups -CH₂-CH₂- or -CH=CH- and Y is a bivalent hydrocarbon group, a siloxane or a combination of both, a = 265-2000, b = 0-249, c = 1-250, with the proviso that b+c ≤ 250,
which is swollen in a solvent.

7. Cosmetic preparation according to Claim 8, **characterized in that** in the siloxane elastomer R₃ is selected from polyethers, pentylene, hexylene, heptylene, octylene, nonylene, decylene, dodecylene, tetradecylene and mixtures thereof.

## Revendications

1. Préparation cosmétique sous la forme d'une émulsion H/E contenant :
a) un ou plusieurs filtres UV,
b) un ou plusieurs gels d'élastomère de silicone,
la préparation contient au moins 10 % en poids, par rapport au poids total de la préparation, de gel d'élastomère de silicone,
c) au moins 1,5 % en poids, par rapport au poids total de la préparation, d'amidon de tapioca,
**caractérisée en ce que** la préparation contient du 4-(tert.-butyl)-4'-méthoxydibenzoyl méthane.

2. Préparation cosmétique selon la revendication 1, **caractérisée en ce que** la préparation contient au moins 5 % en poids, par rapport au poids total de la préparation, de filtres UV.

3. Préparation cosmétique selon l'une quelconque des revendications précédentes, **caractérisée en ce que** la préparation contient un ou plusieurs filtres UV supplémentaires, choisis dans le groupe constitué par les composés de l'acide 2-phénylbenzimidazole-5-sulfonique et/ou leurs sels ; les sels de l'acide phénylène-1,4-bis-(2-benzimidazyl)-3,3'-5,5'-tétrasulfonique ; le 1,4-di-(2-oxo-10-sulfo-3-bornylidène-méthyl)-benzène et ses sels ; les sels de l'acide 4-(2-oxo-3-bornylidène-méthyl)benzène-sulfonique ; les sels de l'acide 2-méthyl-5-(2-oxo-3-bornylidène-méthyl)sulfonique ; le 2,2'-méthylène-bis-(6-(2H-benzotriazol-2-yl)-4-(1,1,3,3-tétraméthylbutyl)-phénol) ; le 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; le 3-(4-méthylbenzylidène)camphre ; le 3-benzylidène-camphre ; le salicylate d'éthylhexyle ; l'acide téréphtalidène-dicamphre-sulfonique ; l'acrylate de 2-éthylhexyl-2-cyano-3,3-diphényle ; l'ester 2-éthylhexylique de l'acide 4-(diméthylamino)-benzoïque ; l'ester amylique de l'acide 4-(diméthylamino)benzoïque ; l'ester di(2-éthylhexylique) de l'acide 4-méthoxybenzalmalonique ; l'ester 2-éthylhexylique de l'acide 4-méthoxycinnamique ; l'ester isoamylique de l'acide 4-méthoxycinnamique ; la 2-hydroxy-4-méthoxybenzophénone, la 2-hydroxy-4-méthoxy-4'-méthylbenzophénone ; la 2,2'-dihydroxy-4-méthoxybenzophénone ; l'ester hexylique de l'acide 2-(4'-diéthylamino-2'-hydroxybenzoyl)-benzoïque ; le salicylate d'homomenthyle ; le 2-hydroxybenzoate de 2-éthylhexyle ; le benzalmalonate de diméthicodiéthyle ; le copolymère de 3-(4-(2,2-bis-éthoxycarbonylvinyl)-phénoxy)propényl)-méthoxysiloxane/diméthylsiloxane ; la dioctylbutylamidotriazone (INCI : diéthylhexyl-butamidotriazone) ; la 2,4-bis-[5-1(diméthyl-propyl)benzoxazol-2-yl-(4-phényl)-imino]-6-(2-éthylhexyl)-imino-1,3,5-triazine avec le n°CAS 288254-16-0 ; l'ester tris-(2-éthylhexylique) de l'acide 4,4',4"-(1,3,5-triazine-2,4,6-triyltriimino)-tris-benzoïque) (également : 2,4,6-tris-[anilino-(p-carbo-2'-éthyl-1'-hexyloxy)]-1,3,5-triazine (INCI : éthylhexyltriazone) ; la 2,4-bis-{[4-(2-éthyl-hexyloxy)-2-hydroxy]-phényl}-6-(4-méthoxyphényl)-1,3,5-triazine ; la 2,4,6-tribiphényl-4-yl-1,3,5-triazine ; la mérocyanine ; le dioxyde de titane ; l'oxyde de zinc.

4. Préparation cosmétique selon l'une quelconque des revendications précédentes, **caractérisée en ce que** l'élastomère de siloxane est fabriqué par le biais du produit d'hydrosilylation de
(1) un polyorganosiloxane comprenant des groupes terminaux alcényle linéaires de formule
M^{vi}ₐDₓD^{vi}_{y}M₂₋ₐ
dans laquelle x est un nombre de 450 à 1 250, y est un nombre de 0 à 200, a est un nombre de 0 à 2, à condition qu'a+y se situe dans la plage allant de 1 à 20, M^{vi} étant défini par
R¹R²R³SiO_{1/2}
R¹ étant un radical hydrocarboné insaturé monovalent de 2 atomes de carbone et R² et R³ étant chacun indépendamment des radicaux hydrocarbonés en C₁ à C₄₀ monovalents, D étant défini par
R⁴R⁵SiO_{2/2}
R⁴ et R⁵ étant chacun indépendamment des radicaux carbonés en C₁₋₄₀ monovalents, D^{vi} étant défini par
D^{vi} = R⁶R⁷SiO_{2/2}
R⁶ étant un radical hydrocarboné insaturé monovalent de 2 à 10 atomes de carbone et R⁷ étant indépendamment un radical hydrocarboné en C₁₋₄₀ monovalent, M étant défini par
M = R⁸R⁹R¹⁰SiO_{1/2}
R⁸, R⁹ et R¹⁰ étant chacun indépendamment un radical hydrocarboné en C₁₋₄₀ monovalent,
et
(2) une résine de formule
(M^{H}_{w}Q_{z})ⱼ
dans laquelle Q a la formule SiO_{4/2} et M^{H} est défini par :
H_{b}R¹¹_{3-b}SiO_{1/2}
R¹¹ étant un radical hydrocarboné en C₁₋₄₀ monovalent, b étant un nombre de 1 à 3, et w et z ayant un rapport de 0,5 à 4,0 et j se situant dans la plage allant de 2,0 à 100,
l'hydrosilylation étant réalisée en présence de
(3) du décaméthylcyclopentasiloxane.

5. Préparation cosmétique selon la revendication 6, **caractérisée en ce que**
R¹ et R⁶ = (CH₂=CH) et
R², R³, R⁴, R⁵, R⁷, R⁸, R⁹ et R¹⁰ = CH₃.

6. Préparation cosmétique selon l'une quelconque des revendications 1 à 5, **caractérisée en ce que** l'élastomère de siloxane présente la structure suivante: 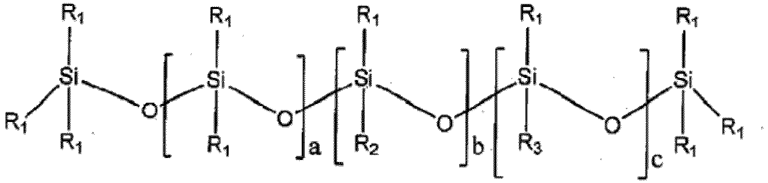 avec
R₁ = radical alkyle en C₁ à C₃₀, R₂ = hydrogène, R₃ = une réticulation transversale -E-Y-E-, l'autre l'extrémité de la réticulation transversale étant reliée avec une deuxième chaîne d'élastomère de silicone, chaque E représentant un groupe bivalent choisi parmi les groupes -CH₂-CH₂- ou -CH=CH-, et Y représentant un groupe hydrocarboné bivalent, un siloxane ou une combinaison des deux, a = 265 à 2 000, b = 0 à 249, c = 1 à 250, à condition que b+c ≤ 250,
qui est gonflée dans un solvant.

7. Préparation cosmétique selon la revendication 8, **caractérisée en ce que**, dans l'élastomère de siloxane, R₃ est choisi parmi les polyéthers, le pentylène, l'hexylène, l'heptylène, l'octylène, le nonylène, le décylène, le dodécylène, le tétradécylène et leurs mélanges.
